# EUROPEAN PATENT APPLICATION

(11) **EP 1 279 383 A1**
(43) Date of publication of application: **29.01.2003**
(21) Application number: 01420161.0
(22) Date of filing: 17.07.2001
(51) Int. Cl.: A61F 5/01

(54) **Ankle brace particularly useful for footdrop**

(71) Applicant: Ormihl, 69100 Villeurbanne (FR)
(72) Inventor: Zick, Ofer, Petach-Tikva (IL)
(74) Representative: Bratel, Gérard

(57) **Abstract**

The ankle brace, particularly useful for persons suffering from footdrop, comprises: a foot section (2) having a curvature conforming to that of the outer surface of the foot on one side of the ankle; a leg section (3) having a curvature conforming to that of the outer surface of the lower leg on the opposite side of the ankle; means for securing the leg section to the wearer's leg; and pivotal mounting means (4) connecting said foot and leg sections together at the ankle. said pivotal mounting means includes spring means (44,45) biasing said foot section to be substantially perpendicular to said leg section, and limit means (21a,31a) for limiting the pivotal movement of the foot section when pivoted to a larger angle than 90°.

## Description

The present invention relates to an ankle brace. The invention is particularly useful when embodied as an ankle brace for persons suffering from footdrop, and is therefore described with respect to such an application.

Footdrop is a condition in which the toes of the foot drag and the foot hangs. As a result, the arch of the foot sinks down, and the inner edge of the foot rests upon the ground as the person walks. This condition is caused by weakness or paralysis of the muscles on the side of the shinbone, e.g. by poliomyelitis, polyneuritis, or one of the muscular dystrophies.

Various types of braces have been devised for use by persons suffering from footdrop. One type, widely used in poliomyelitis, is secured to the outside of the wearer's shoe; this type is unsightly in appearance and cumbersome to apply and to use. Another type which fits within the wearer's shoe is of a rigid construction and holds the foot perpendicular to the leg; this type does not permit the ankle to pivot, which makes it difficult to use, particularly when climbing stairs, besides being conspicuous when used.

An object of the present invention is to provide a novel ankle brace particularly useful for persons suffering from footdrop and having advantages in the above respects.

According to the present invention, there is provided an ankle brace particularly useful for persons suffering from footdrop, comprising: a foot section having a curvature conforming to that of the outer surface of the foot-on one side of the ankle; a leg section having a curvature conforming to that of the outer surface of the lower leg on the opposite side of the ankle; means for securing the leg section to the wearer's leg; and pivotal mounting means connecting the foot and leg sections together at the ankle; the pivotal mounting means including spring means biassing the foot section to be substantially perpendicular to the leg section, and limit means for limiting the pivotal movement of the foot section when pivoted to a larger angle than 90°.

According to further features in the preferred embodiment of the invention described below, the securing means comprises straps for securing the leg section to the wearer's leg. When the ankle brace is used within the wearer's shoe, the shoe itself secures the foot section to the wearer's ankle. However, the ankle brace could be used without a shoe, in which case the brace would preferably include straps for securing the foot section to the wearer's foot.

According to further features in the described preferred embodiment, the limit means limits the pivotal movement of the foot not only to a larger angle from perpendicularity, but also to a smaller angle. Preferably, the larger angle is about 120°, and the smaller angle is about 80°, to thereby permit the wearer's foot to simulate all the natural movements of the foot.

According to an additional feature in the described preferred embodiment, the ankle brace further includes a liner to underlie the leg section so as to be between it and the wearer's leg when the brace is attached to the wearer's ankle; the liner also has a curvature conforming to the outer surface of the wearer's leg, and is of a larger surface area than that of the leg section. The provision of such a liner cushions the force and spreads it for a greater area over the shinbone, thereby making the brace more comfortable in use. In addition, the liner is removable to permit cleaning and replacement when necessary. A similar liner may also be provided in the foot section.

According to still further features in the described preferred embodiment, the pivotal mounting comprises a first plate fixed to the foot section at each of its opposite sides; a second plate fixed to the leg section at each of its opposite sides; and a pivot pin pivotally mounting the two plates to each other. In addition, the limit means comprises end surfaces of the foot and leg sections which are configured to abut each other at the limit positions. Further, the spring means comprises a U-shaped spring inserted on each side of each of the pivotal mountings, each spring having: a first leg bearing against the end surface of the respective side of the foot section; a second leg bearing against the end surface of the respective side of the leg section; and an elastic juncture between the two legs.

As will be more apparent from the description below, an ankle brace constructed in accordance with the foregoing features permits a user suffering from footdrop to walk quite naturally. Moreover, it is relatively inconspicuous when used, is convenient to apply, and relatively comfortable when worn.

Further features and advantages of the invention will be apparent from the description below.

The invention is herein described, by way of example only, with reference to the accompanying drawings, wherein:

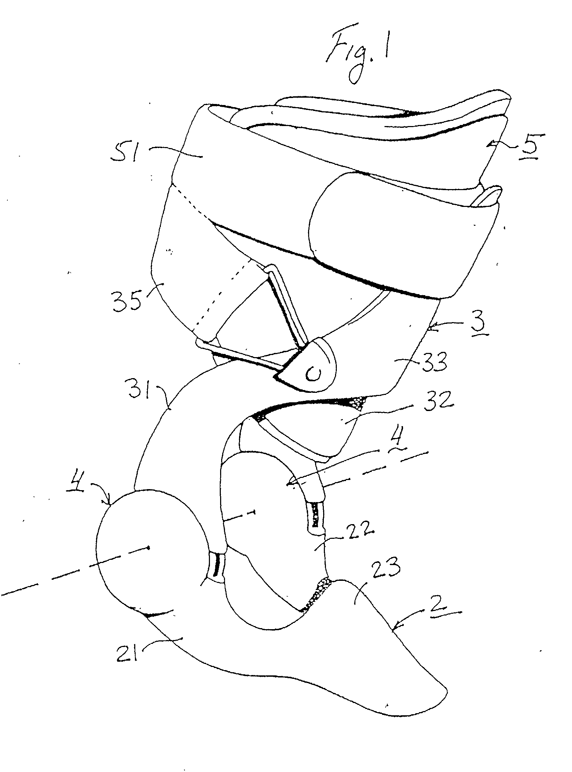

Fig. 1 is a perspective view illustrating one form of ankle brace constructed in accordance with the present invention;

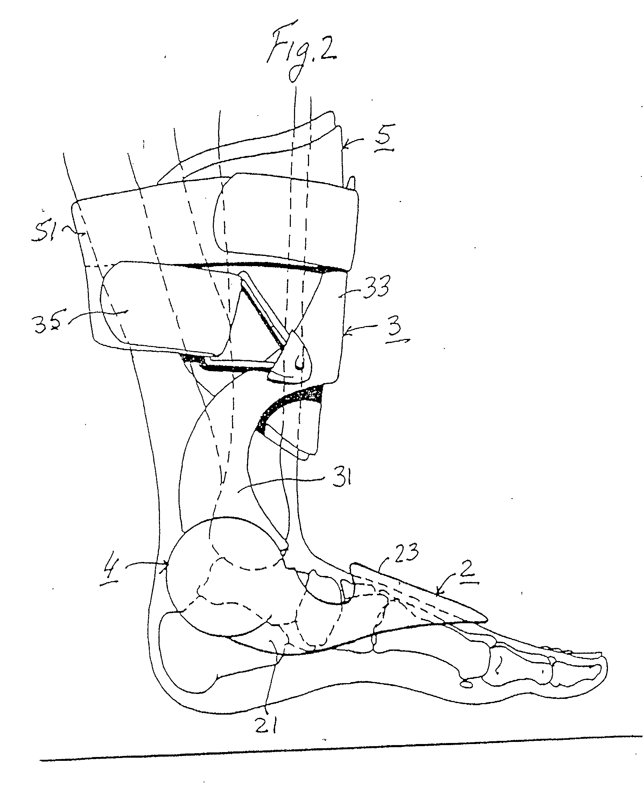

Fig. 2 is a side elevational view of the ankle brace of Fig. 1 as applied to a wearer's foot;
Fig. 3 illustrates the ankle brace of Figs. 1 and 2 when used by a person wearing a shoe;
Fig. 4 illustrates five movements of the foot during natural walking;
Fig. 5 illustrates the ankle brace of Figs. 1 and 2, and particularly the limits of the pivotal movements permitted by the brace; and
Figs. 6a, 6b, and 6c illustrate the construction of the pivotal mountings and the limits of the permitted pivotal movements shown in Fig. 5.

The ankle brace illustrated in the drawings is particularly useful for persons suffering from footdrop and aids such persons to walk in a relatively normal manner. The brace comprises a foot section 2 having a curvature conforming to that of the outer surface of the foot on one side of the wearer's ankle, a leg section 3 having a curvature conforming to that of the outer surface of the lower leg on the opposite side of the wearer's ankle, and a pivotal mounting, generally designated 4, on each side of the brace and pivotally connecting the foot and leg sections together at the ankle.

Each pivotal mounting 4 includes a spring which biasses the foot section normally to be substantially perpendicular to the leg section, but permits the foot section to pivot in both directions away from perpendicularity. Each pivotal mounting, however, includes limits for limiting the pivotal movements of the foot section in both directions, i.e. in the direction tending to enlarge the normal 90° angle, or in the opposite direction to reduce that angle.

The ankle brace illustrated in the drawings further includes a liner 5 to underlie the leg section 3 of the brace. It may also include a liner (not shown) to underlie the foot section 2 of the brace. The liner is preferably made of cushioning material, such as sponge rubber. It has a curvature conforming to that of the outer surface of the wearer's leg, and is of a larger surface area than that of the leg section 3. The same would apply if a liner for the foot section is also included.

The foot section 2 includes a pair of sides 21, 22 constructed to extend longitudinally, and on opposite sides, of the wearer's foot. The two sides 21, 22 are joined at one end by a curved arch 23 to extend transversely across the upper surface of the wearer's foot. Arch 23 is curved to conform to the curvature of the upper surface of the wearer's foot and is of a relatively large surface area so as to spread the force applied by the brace to the wearer's foot. The opposite ends of the sides 21, 22 are connected to the leg section 3 by the pivotal mountings 4.

The leg section 3 is similarly constructed of two sides 31, 32 adapted to extend longitudinally of the wearer's lower leg and joined at one end by a curved arch 33 to extend transversely across the outer surface of the lower leg. The arch 33 is curved to conform to the outer curvature of the wearer's leg (i.e. the lower part of the shinbone), and is similarly of a relatively large surface area to spread the force applied by the ankle brace to the wearer's leg. The opposite ends of the sides 31, 32 are connected to the two sides 21, 22 of the foot section 2 by the pivotal mountings 4.

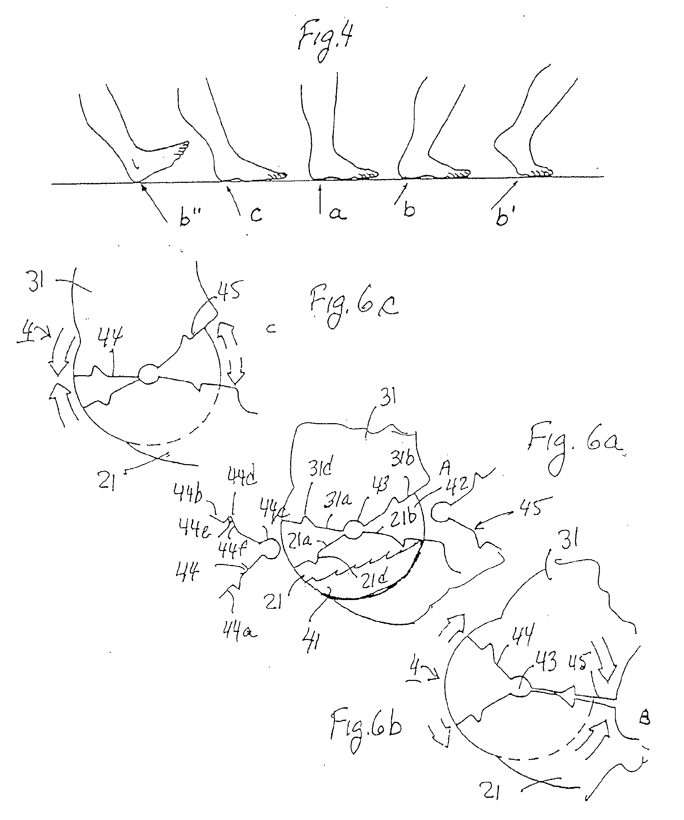

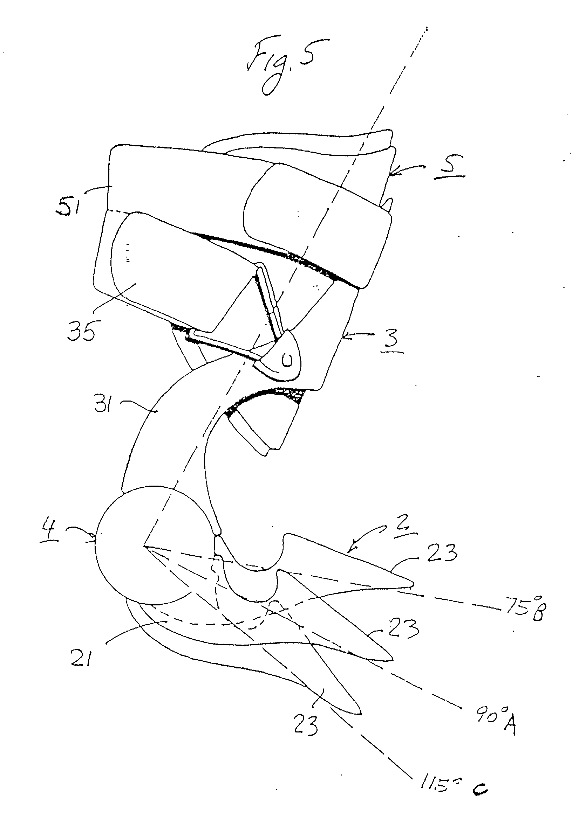

Fig. 6a illustrates the construction of each of the pivotal mountings 4. Thus, each pivotal mounting 4 includes a first circular plate 41 fixed to the respective side (e.g. 21) of the foot section 2, a second circular plate 42 fixed to the respective side (e.g. 31) of the leg section 3, and a pivot pin 43 pivotally mounting the two plates to each other.

Each pivotal mounting 4 further includes two U-shaped springs 44, 45, one on each side of the pivot pin 43. The two springs 44, 45 are of similar construction, of a U-shaped configuration. Each spring has a first leg (e.g. 44a) bearing against the end surface of the foot section side 21, a second leg (e.g. 44b) bearing against the end surface of the leg section side 31, and an elastic juncture (e.g. 44c) of circular configuration receivable on the pivot pin 43.

Each leg is preferably formed with an external bend (e.g. 44d) receivable within a recess (e.g. recesses 21d, 31d in surfaces 21a, 31a, respectively) for retaining the spring in position between these surfaces. As shown, each bend 44d includes a right-angle face 44e facing the outer side of the pivotal mounting to prevent accidental removal of the spring, and an inclined face 44f facing the pivot pin 43 to facilitate the insertion of the spring.

As shown particularly in Fig. 6a, the confronting ends of the foot section side 21 and the leg section side 31 are configured to permit a predetermined pivotal movement between the two sections before these surfaces abut. Springs 44, 45 normally bias the foot section 2 to be substantially perpendicular to the leg section 3. The confronting surfaces (21a, 31a, Fig. 6a) at the left of pivot pin 43 will thus limit the pivotal movement of the foot section 2 in the direction of increase in the angle between it and the leg section 3, whereas the confronting surfaces 21b, 31b at the right of pivot pin 43 will limit the pivotal movement of the foot section 2 in the direction of decrease in this angle.

The foregoing arrangement enables the wearer's leg to more closely simulate a natural walking movement, as illustrated in Fig. 4. Thus, when the wearer is standing, i.e. in the "mid-stand" position as shown at "a", Fig. 4, the foot is substantially perpendicular to the leg. This is the condition of the pivotal mounting illustrated in Fig. 6a, wherein the two springs 44 and 45 bias the foot section 2 to be substantially perpendicular to the leg section 3 of the ankle brace.

As the wearer begins a step, the leg first pivots with respect to the ankle in the direction to decrease the angle with the ground. This pivotal movement is first taken up by pivoting of the foot and is maximum at position "b", which is the beginning of the "toe-off" position. In this position, the angle between the leg and foot is approximately 75°. This angle is permitted by the confronting faces 21b, 31b illustrated in Fig. 6a, which abut at the beginning of the "toe-off" position illustrated in Fig. 5b.

As the foot leaves the toe-off position, the angle between the foot and leg moves toward perpendicularity, and the toes now bend as the weight is shifted to the other foot. At the end of the "toe-off" (position "b"), the other foot, starting with the foot perpendicular to the leg (position "b"), pivots at the ankle to increase its angle with respect to the leg as that foot moves to the "heel-strike" position illustrated at "c" in Fig. 4.

In a normal foot, the foot muscles at the ankle-retain the foot substantially perpendicular to the leg while the foot reaches the heel-strike position "c". However, in a footdrop condition, this is not the case, and the foot drops so that the angle between it and the leg is substantially increased; that is, the toes drag and the foot hangs because of weakness or paralysis of the muscles on the side of the shinbone. The use of the ankle brace illustrated in the drawings, however, prevents the foot from dropping, since the confronting surfaces 21a, 31a of the foot and leg sections at each of the pivotal mountings 4 abut and thereby limit the pivotal movement of the foot in this direction, as shown in Fig. 6c.

Preferably, as shown in Fig. 5, the foot section is normally at a 90° angle with respect to the leg section (position A); the confronting surfaces 21a, 31a are such as to abut at about 115° (position C), and confronting surfaces 21b, 31b are such as to abut at about 750° (position B).

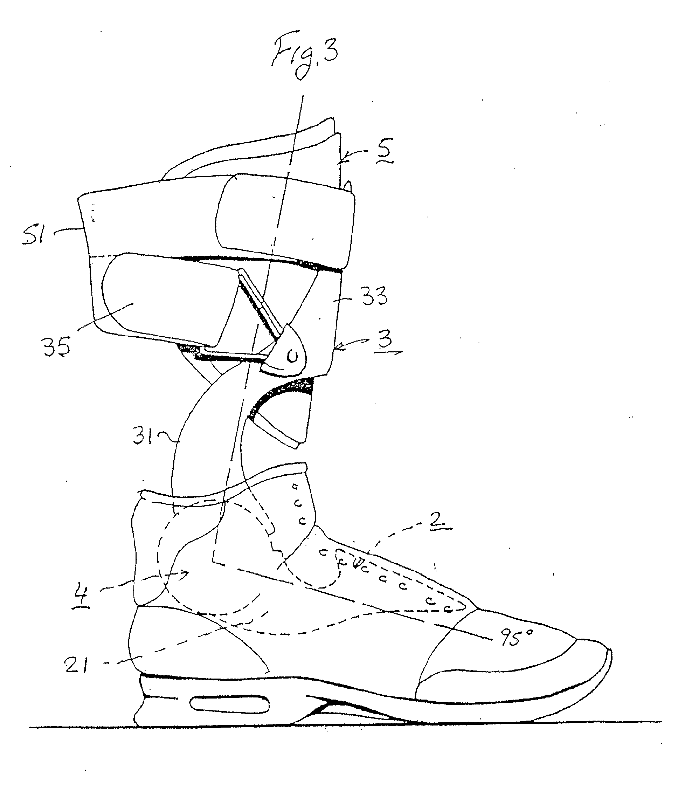

The leg section 3 is attachable to the wearer's leg by bands 35. Similar bands may be provided for the foot section 2, to permit the ankle brace to be worn even without a shoe or perhaps with a soft slipper. If the ankle brace is worn with a shoe, the foot bands may be omitted, since the shoe will fix the foot section 2 to the wearer's foot. As also shown in Fig. 3, when the shoe includes a heel, the normal angle between the foot and leg sections is generally increased from the usual 90° to about 95°.

Liner 5 may be similarly provided with one or more bands 51 to fix the liner to the wearer's leg.

The foot section 2 and leg section 3 of the brace are preferably made of rigid, strong, relatively light-weight plastic material. Preferably, fiber carbon material is used for this purpose because of its high strength for its low weight, but other materials may also be used.

While the invention has been described with respect to one preferred embodiment, it will be appreciated that many other variations, modifications and applications of the invention may be made.

## Claims

1. An ankle brace particularly useful for persons suffering from footdrop, comprising:
a foot section having a curvature conforming to that of the outer surface of the foot on one side of the ankle;
a leg section having a curvature conforming to that of the outer surface of the lower leg on the opposite side of the ankle;
means for securing the leg section to the wearer's leg;
and pivotal mounting means connecting said foot and leg sections together at the ankle;
said pivotal mounting means including spring means biassing said foot section to be substantially perpendicular to said leg section, and limit means for limiting the pivotal movement of the foot section when pivoted to a larger angle than 90°.

2. The ankle brace according to Claim 1, wherein said securing means comprises straps for securing said leg section to the wearer's leg.

3. The ankle brace according to Claim 2, further including straps for securing said foot section to the wearer's foot.

4. The ankle brace according to any one of Claims 1-3, wherein said larger angle is about 115°.

5. The ankle brace according to any one of Claims 1-4, wherein said limit means also limits the pivotal movement of the foot section when pivoted to a smaller angle than 90°.

6. The ankle brace according to Claim 5, wherein said smaller angle is about 75°.

7. The ankle brace according to any one of Claims 1-6, further including a liner to underlie said leg section so as to be between it and the wearer's leg when the brace is attached to the wearer's ankle; said liner being made of a cushioning material, also having a curvature conforming to the outer surface of the wearer's leg, and being of a larger surface area than that of said leg section.

8. The ankle brace according to Claim 7, wherein said liner also includes straps for attaching it to the wearer's leg.

9. The ankle brace according to any one of Claims 1-8, further including a liner to underlie said foot section so as to be between it and the wearer's foot when the brace is attached to the wearer.

10. The ankle brace according to Claim 9, wherein said foot liner includes straps for attaching same to the wearer's foot.

11. The ankle brace according to any one of Claims 1-10, wherein said foot section includes two sides to extend longitudinally of the foot on opposite sides thereof and joined at one end by a curved arch to extend transversely across the upper surface of the foot; said pivotal mounting means including a pivotal mounting between the opposite end of each of said sides and said leg section.

12. The ankle brace according to Claim 11, wherein said leg section includes two sides to extend longitudinally of the lower leg on opposite sides thereof and joined at one end by a curved arch to extend transversely across the outer surface of the lower leg; each of said pivotal mountings being between the end of each of said sides of the leg section and of said foot section.

13. The ankle brace according to Claim 12, wherein said pivotal mounting comprises:
a first plate fixed to said foot section at each of its opposite sides;
a second plate fixed to said leg section at each of its opposite sides;
and a pivot pin pivotally mounting said two plates to each other.

14. The ankle brace according to Claim 13, wherein said first and second plates are circular plates and are pivotally mounted to each other at their centers.

15. The ankle brace according to either of Claims 13 or 14, wherein said limit means comprises end surfaces of said foot and leg sections which are configured to abut each other at the limit positions.

16. The ankle brace according to Claim 15, wherein said spring means comprises a U-shaped spring inserted on each side of each of said pivotal mountings, each spring having:
a first leg bearing against the end surface of the respective side of the foot section;
a second leg bearing against the end surface of the respective side of the leg section;
and an elastic juncture between the two legs.

17. The ankle brace according to Claim 16, wherein said elastic juncture of each spring is of circular configuration and is received on the pivot pin of the respective pivotal mounting.

18. The ankle brace according to either of Claims 16 or 17, wherein each leg of each spring is formed with an external bend received in a recess formed in the end surface of the respective section.

19. The ankle brace according to Claim 18, wherein said external bend formed on each leg of each spring includes a right-angle face to face the outer side of the pivotal mounting and thereby to prevent the inadvertent removal of the spring, and an inclined face facing the pivotal pin.

20. An ankle brace particularly useful for persons suffering from footdrop, substantially as described with reference to and as illustrated in the accompanying drawings.
